# EUROPÄISCHE PATENTSCHRIFT

(11) **EP 0 984 945 B1**
(45) Veröffentlichungstag und Bekanntmachung des Hinweises auf die Patenterteilung: **27.04.2005**
(21) Anmeldenummer: 98930691.5
(22) Anmeldetag: 14.05.1998
(51) Int. Cl.: C07D 239/54, A01N 43/54

(54) **SUBSTITUIERTE IMINOALKOXY-PHENYLURACILE, DEREN HERSTELLUNG UND DEREN VERWENDUNG ALS HERBIZIDE**
SUBSTITUTED IMINOALKOXY-PHENYLURACILS, THE PRODUCTION AND USE THEREOF AS HERBICIDES
IMINOALCOXY-PHENYLURACILES SUBSTITUES, LEUR FABRICATION ET LEUR UTILISATION COMME HERBICIDES

(30) Priorität: 27.05.1997 DE 19722031
(43) Veröffentlichungstag der Anmeldung: 15.03.2000
(73) Patentinhaber: Bayer CropScience AG, 40789 Monheim (DE)
(72) Erfinder: DREWES, Mark, Wilhelm, D-40764 Langenfeld (DE); ANDREE, Roland, D-40764 Langenfeld (DE); DOLLINGER, Markus, D-51381 Leverkusen (DE)
(86) Internationale Anmeldenummer: PCT/EP1998/002861
(87) Internationale Veröffentlichungsnummer: WO 1998/054155

(56) Entgegenhaltungen:
- EP-A- 0 255 047
- WO-A-93/11669
- WO-A-95/06641
- WO-A-95/25725
- WO-A-96/24590
- WO-A-97/01541
- WO-A-97/05117
- DE-A- 19 524 617

## Beschreibung

Die Erfindung betrifft neue substituierte Iminoalkoxy-phenyluracile, Verfahren zu ihrer Herstellung und ihre Verwendung als Herbizide.

Bestimmte substituierte Aryluracile, wie z.B. die Verbindung 3-[4-Chlor-2-fluor-5-(2-oxo-propoxy)-phenyl]-1-methyl-6-trifluormethyl-(1H,3H)-pyrimidin-2,4-dion, sind bereits aus der (Patent-)Literatur bekannt (vgl. EP 255 047 A). Weitere Verbindungen dieses Typs wurden auch bereits in WO 93/11669 A, WO 97/01541 A, WO 95/06641 A, WO 95/25725 A, WO 96/24590 A, WO 97/0517 A und DE 195 24 167 A beschrieben. Diese Verbindungen haben jedoch bisher keine besondere Bedeutung erlangt.

Es wurden nun neue substituierte Iminoalkoxy-phenyluracile der allgemeinen Formel (I) 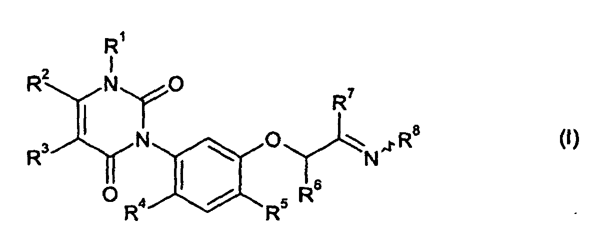 in welcher
- R¹: für Wasserstoff, Amino oder gegebenenfalls durch Cyano, Fluor, Chlor oder C₁-C₄-Alkoxy substituiertes C₁-C₆-Alkyl steht,
- R²: für Carboxy, Cyano, Carbamoyl, Thiocarbamoyl oder für jeweils gegebenenfalls durch Cyano, Fluor, Chlor oder C₁-C₄-Alkoxy substituiertes C₁-C₄-Alkyl oder C₁-C₄-Alkoxycarbonyl steht,
- R³: für Wasserstoff, Fluor, Chlor, Brom oder gegebenenfalls durch Fluor oder Chlor substituiertes C₁-C₄-Alkyl steht,
- R⁴: für Wasserstoff, Cyano, Thiocarbamoyl, Fluor, Chlor oder Brom steht,
- R⁵: für Cyano, Thiocarbamoyl, Fluor, Chlor oder Brom steht,
- R⁶: für Wasserstoff oder gegebenenfalls durch Cyano, Fluor, Chlor oder C₁-C₄-Alkoxy substituiertes C₁-C₆-Alkyl steht,
- R⁷: für gegebenenfalls durch Cyano, Fluor, Chlor oder C₁-C₄-Alkoxy substituiertes C₁-C₆-Alkyl steht, und
- R⁸: für Hydroxy, für Amino oder für jeweils gegebenenfalls durch Carboxy, Cyano, Halogen, C₁-C₄-Alkoxy oder C₁-C₄-Alkoxy-carbonyl substituiertes Alkyl, Alkoxy, Alkylamino, Dialkylamino, Alkylcarbonylamino, Alkoxycarbonylamino oder Alkylsulfonylamino mit jeweils 1 bis 6 Kohlenstoffatomen steht,
- R⁸: weiterhin für jeweils gegebenenfalls durch Halogen substituiertes Alkenyl, Alkinyl, Alkenyloxy oder Alkinyloxy mit jeweils 3 bis 6 Kohlenstoffatomen steht, oder
- R⁸: weiterhin für jeweils gegebenenfalls durch Nitro, Cyano, Carboxy, Halogen, C₁-C₄-Alkyl, C₁-C₄-Halogenalkyl, C₁-C₄-Alkoxy oder C₁-C₄-Halogenalkoxy substituiertes Aryl, Aryloxy, Arylamino, Arylcarbonylamino, Arylsulfonylamino, Arylalkyl, Arylalkylamino, Arylalkylcarbonylamino oder Arylalkylsulfonylamino mit jeweils 6 oder 10 Kohlenstoffatomen im Arylteil und gegebenenfalls 1 bis 4 Kohlenstoffatomen im Alkylteil steht,
gefunden.

Man erhält die neuen substituierten Iminoalkoxy-phenyluracile der allgemeinen Formel (I), wenn man substituierte Oxoalkoxy-phenyluracile der allgemeinen Formel (II) 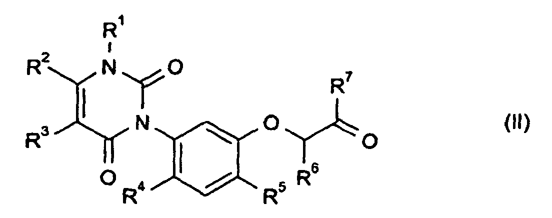 in welcher
- R¹, R², R³, R⁴, R⁵, R⁶ und R⁷: die oben angegebenen Bedeutungen haben,
mit Aminoverbindungen der allgemeinen Formel (III)

H₂N-R⁸ (III)

in welcher
- R⁸: die oben angegebene Bedeutung hat,
- oder mit Säureaddukten von Verbindungen der allgemeinen Formel (III) -
gegebenenfalls in Gegenwart eines Reaktionshilfsmittels und gegebenenfalls in Gegenwart eines Verdünnungsmittels umsetzt und gegebenenfalls im Anschluß daran im Rahmen der Substituentendefinition auf übliche Weise elektrophile oder nucleophile Substitutionsreaktionen bzw. Additionsreaktionen durchführt.

Die Verbindungen der allgemeinen Formel (I) können nach üblichen Methoden in andere Verbindungen der allgemeinen Formel (I) gemäß obiger Definition umgewandelt werden, beispielsweise durch Aminierung bzw. Alkylierung (z.B. R¹: H → NH₂, H → CH₃), Umsetzung mit Dicyan bzw. Hydrogensulfid (z.B. R⁵: Br → CN, CN → CSNH₂, vgl. die Herstellungsbeispiele).

Die neuen substituierten Iminoalkoxy-phenyluracile der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- R¹: für Wasserstoff, für Amino oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
- R²: für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, Methoxycarbonyl oder Ethoxycarbonyl steht,
- R³: für Wasserstoff, Fluor, Chlor, Brom oder für jeweils gegebenenfalls durch Fluor oder Chlor substituiertes Methyl oder Ethyl steht,
- R⁴: für Wasserstoff, Fluor oder Chlor steht,
- R⁵: für Cyano, Thiocarbamoyl, Chlor oder Brom steht,
- R⁶: für Wasserstoff oder für jeweils gegebenenfalls durch Cyano, Fluor, Chlor, Methoxy oder Ethoxy substituiertes Methyl, Ethyl, n- oder i-Propyl, n-, i-, s-oder t-Butyl steht,
- R⁷: für jeweils gegebenenfalls durch Cyano, Fluor, Chlor, Methoxy oder Ethoxy substituiertes Methyl, Ethyl, n- oder i-Propyl, n-, i-, s- oder t-Butyl steht, und
- R⁸: für Hydroxy, für Amino, oder für jeweils gegebenenfalls durch Carboxy, Cyano, Fluor, Chlor, Methoxy, Ethoxy, n- oder i-Propoxy, Methoxycarbonyl, Ethoxycarbonyl, n- oder i-Propoxycarbonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Acetylamino, Propionylamino, Methoxycarbonylamino, Ethoxycarbonylamino, Methylsulfonylamino oder Ethylsulfonylamino steht, oder
- R⁸: weiterhin für jeweils gegebenenfalls durch Fluor, Chlor oder Brom substituiertes Propenyl, Butenyl, Propinyl, Butinyl, Propenyloxy, Butenyloxy, Propinyloxy oder Butinyloxy steht, oder
- R⁸: weiterhin für jeweils gegebenenfalls durch Nitro, Cyano, Carboxy, Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s- oder t-Butoxy, Difluormethoxy, Trifluormethoxy substituiertes Phenyl, Phenoxy, Phenylamino, Benzoylamino, Phenylsulfonylamino, Benzyl, Benzylamino, Benzylcarbonylamino oder Benzylsulfonylamino steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Verwendet man beispielsweise 3-[4-Chlor-2-fluor-5-(2-oxo-propoxy)-phenyl]-1-methyl-6-trifluormethyl-(1H,3H)-pyrimidin-2,4-dion und O-Methylhydroxylamin als Ausgangsstoffe, so kann der Reaktionsablauf beim erfindungsgemäßen Herstellungsverfahren durch das folgende Formelschema skizziert werden:

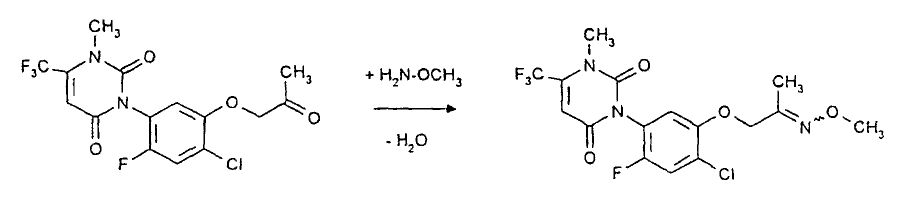

Die beim erfindungsgemäßen Verfahren zur Herstellung von Verbindungen der Formel (I) als Ausgangsstoffe zu verwendenden substituierten Oxoalkoxy-phenyluracile sind durch die Formel (II) allgemein definiert. In der Formel (II) haben R¹ , R², R³, R⁴, R⁵, R⁶ und R⁷ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ , R², R³, R⁴, R⁵, R⁶ und R⁷ angegeben wurden.

Die Ausgangsstoffe der allgemeinen Formel (II) sind bekannt und/oder können nach bekannten Verfahren hergestellt werden (vgl. EP 255047, Herstellungsbeispiele).

Die beim erfindungsgemäßen Verfahren weiter als Ausgangsstoffe zu verwendenden Aminoverbindungen sind durch die Formel (III) allgemein definiert. In der Formel (III) hat R⁸ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁸ angegeben wurde. Es können vorzugsweise auch Addukte von Verbindungen der Formel (III) mit starken Säuren, insbesondere mit Mineralsäuren, wie z.B. Hydrogenchlorid, Hydrogenbromid oder Schwefelsäure, beim erfindungsgemäßen Verfahren eingesetzt werden.

Die Ausgangsstoffe der allgemeinen Formel (III) sind bekannte Synthesechemikalien.

Als Reaktionshilfsmittel für das erfindungsgemäße Verfahren zur Herstellung von Verbindungen der Formel (I) kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calciumhydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon; Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Asulam, Atrazine, Azimsulfuron, Benazolin, Benfuresate, Bensulfuron(-methyl), Bentazon, Benzofenap, Benzoylprop(-ethyl), Bialaphos, Bifenox, Bromobutide, Bromofenoxim, Bromoxynil, Butachlor, Butylate, Cafenstrole, Carbetamide, Chlomethoxyfen, Chloramben, Chloridazon, Chlorimuron(ethyl), Chlornitrofen, Chlorsulfuron, Chlortoluron, Cinmethylin, Cinosulfuron, Clethodim, Clodinafop(-propargyl), Clomazone, Clopyralid, Clopyrasulfuron, Cloransulam(-methyl), Cumyluron, Cyanazine, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Difenzoquat, Diflufenican, Dimefuron, Dimepiperate, Dimethachlor, Dimethametryn, Dimethenamid, Dinitramine, Diphenamid, Diquat, Dithiopyr, Diuron, Dymron, EPTC, Esprocarb, Ethalfluralin, Ethametsulfuron(-methyl), Ethofumesate, Ethoxyfen, Etobenzanid, Fenoxaprop(-ethyl), Flamprop(-isopropyl), Flamprop(-isopropyl-L), Flamprop(-methyl), Flazasulfuron, Fluazifop(-butyl), Flumetsulam, Flumiclorac(-pentyl), Flumioxazin, Flumipropyn, Fluometuron, Fluorochloridone, Fluoroglycofen(-ethyl), Flupoxam, Flupropacil, Flurenol, Fluridone, Fluroxypyr, Flurprimidol, Flurtamone, Fomesafen, Glufosinate(-ammonium), Glyphosate(-isopropylammonium), Halosafen, Haloxyfop(-ethoxyethyl), Hexazinone, Imazamethabenz(methyl), Imazamethapyr, Imazamox, Imazapyr, Imazaquin, Imazethapyr, Imazosulfuron, Ioxynil, Isopropalin, Isoproturon, Isoxaben, Isoxaflutole, Isoxapyrifop, Lactofen, Lenacil, Linuron, MCPA, MCPP, Mefenacet, Metamitron, Metazachlor, Methabenzthiazuron, Metobenzuron, Metobromuron, Metolachlor, Metosulam, Metoxuron, Metribuzin, Metsulfuron(-methyl), Molinate, Monolinuron, Naproanilide, Napropamide, Neburon, Nicosulfuron, Norflurazon Orbencarb, Oryzalin, Oxadiazon, Oxyfluorfen, Paraquat, Pendimethalin, Phenmedipham, Piperophos, Pretilachlor, Primisulfuron(-methyl), Prometryn, Propachlor, Propanil, Propaquizafop, Propyzamide, Prosulfocarb, Prosulfuron, Pyrazolate, Pyrazosulfuron(-ethyl), Pyrazoxyfen, Pyributicarb, Pyridate, Pyrithiobac(-sodium), Quinchlorac, Quinmerac, Quizalofop(ethyl), Quizalofop(-p-tefuryl), Rimsulfuron, Sethoxydim, Simazine, Simetryn, Sulcotrione, Sulfentrazone, Sulfometuron(-methyl), Sulfosate, Tebutam, Tebuthiuron, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl, vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

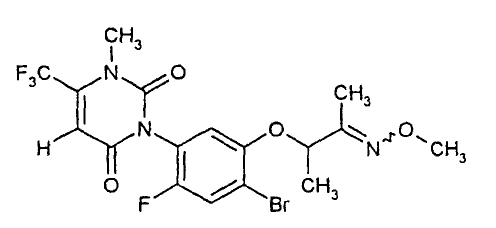

Eine Mischung aus 1,5 g (3,31 mMol) 3-[4-Brom-2-fluor-5-(1-methyl-2-oxopropoxy)-phenyl]-1-methyl-6-trifluormethyl-(1H,3H)-pyrimidin-2,4-dion, 0,30 g (3,31 mMol) O-Methyl-hydroxylamin, 0,30 g Natriumacetat und 20 ml Ethanol wird eine Stunde bei Raumtemperatur (ca. 20°C) gerührt und dann im Wasserstrahlvakuum eingeengt. Der Rückstand wird in Wasser aufgenommen, mit 1N-Salzsäure auf einen pH-Wert zwischen 2 und 3 eingestellt und mit Diethylether geschüttelt. Die organische Phase wird mit Wasser gewaschen, mit Natriumsulfat getrocknet und filtriert. Vom Filtrat wird dann das Lösungsmittel im Wasserstrahlvakuum sorgfältig abdestilliert.

Man erhält 1,5 g (94 % der Theorie) 3-[4-Brom-2-fluor-5-(1-methyl-2-methoximinopropoxy)-phenyl]-1-methyl-6-trifluormethyl-(1H,3H)-pyrimidin-2,4-dion als amorphen Rückstand.

### Beispiel 2

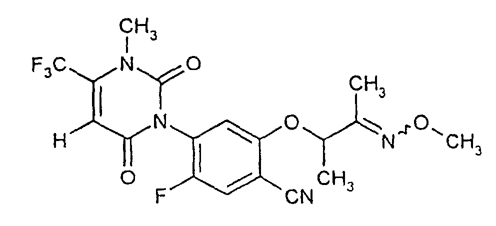

Eine Mischung aus 0,90 g (1,86 mMol) ) 3-[4-Brom-2-fluor-5-(1-methyl-2-methoximino-propoxy)-phenyl]-1-methyl-6-trifluormethyl-(1H,3H)-pyrimidin-2,4-dion, 0,11 g Zink(II)-cyanid, 0,08 g Tetrakis-(triphenylphosphin)-Palladium(0) und 10 ml N,N-Dimethyl-formamid wird unter einer Argon-Atmosphäre 8 Stunden lang auf 80°C bis 90°C erhitzt. Nach Zugabe von weiteren 0,1 g Zink(II)-cyanid und 0,07 g Tetrakis-(triphenylphosphin)-Palladium(0) wird die Mischung weiter 30 Stunden lang auf 80°C bis 90°C erhitzt. Dann wird die Mischung mit 1N-Salzsäure und Essigsäureethylester geschüttelt, die organische Phase abgetrennt und im Wasserstrahlvakuum eingeengt. Der Rückstand wird säulenchromatografisch (Kieselgel, Hexan/Essigsäureethylester, Vol. 4/1) gereinigt.

Man erhält 0,40 g (50 % der Theorie) 3-[4-Cyano-2-fluor-5-(1-methyl-2-methoximino-propoxy)-phenyl]-1-methyl-6-trifluormethyl-(1H,3H)-pyrimidin-2,4-dion als amorphes Produkt.

Analog zu den Herstellungsbeispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

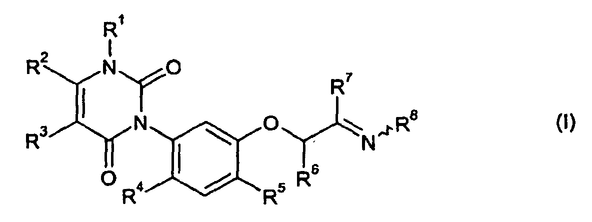

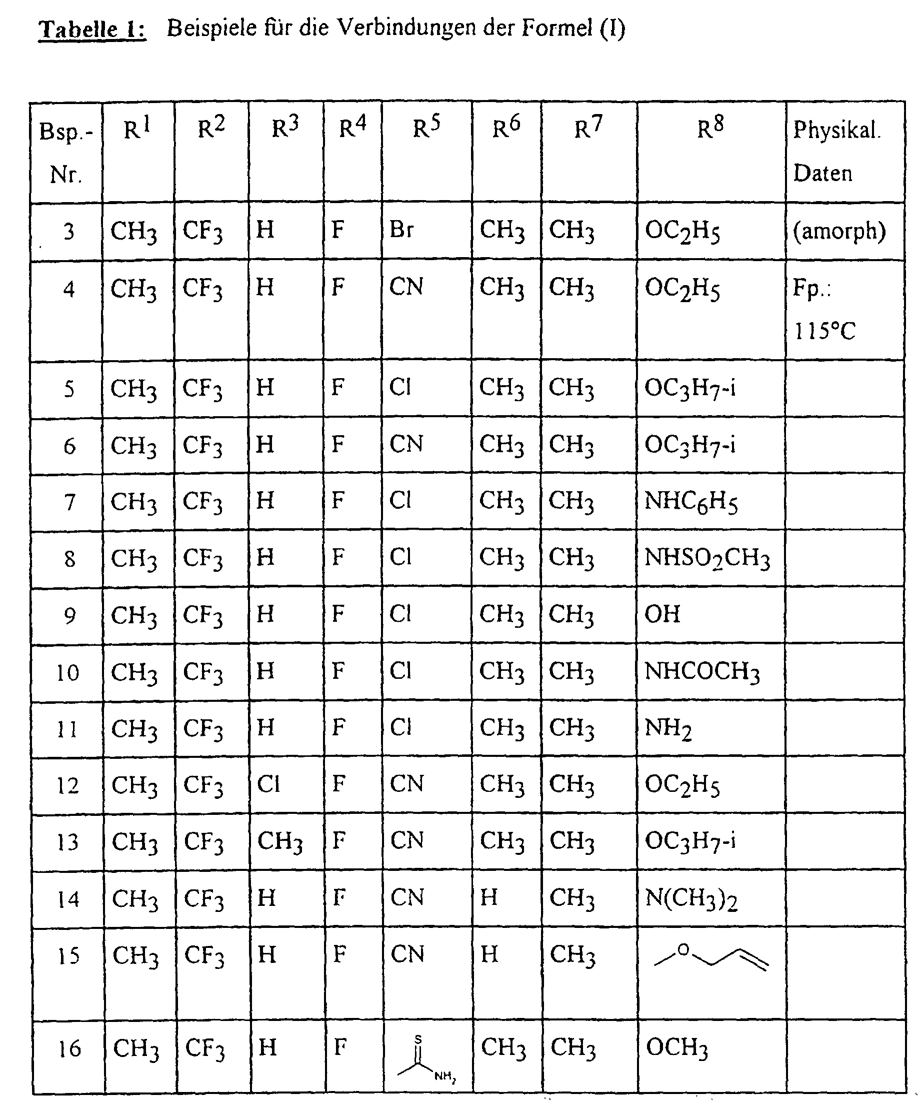

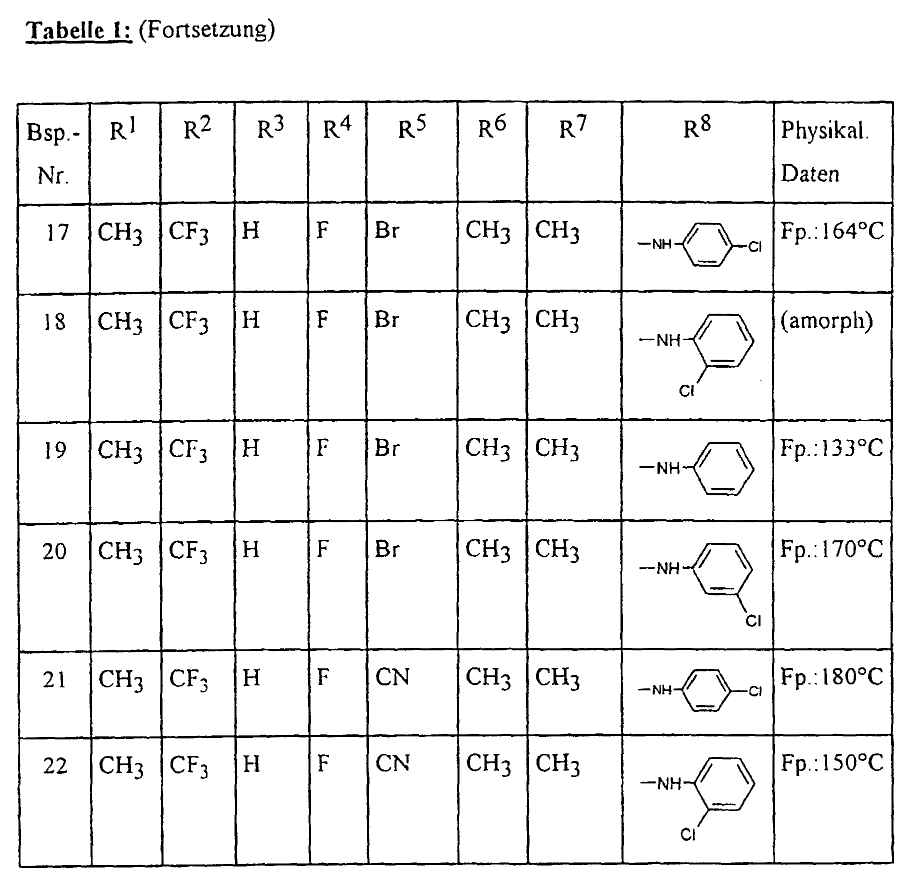

### Ausgangsstoffe der Formel (II):

### Beispiel II-1

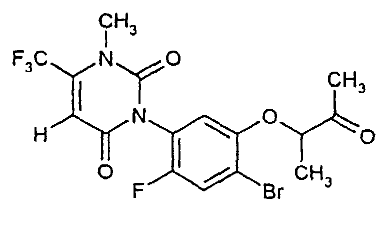

Eine Mischung aus 2,8 g (6,38 mMol) 3-[4-Brom-2-fluor-5-(1-methyl-2-oxo-propoxy)-phenyl]-6-trifluormethyl-(1H,3H)-pyrimidin-2,4-dion, 0,10 g (8 mMol) Dimethylsulfat, 1 g Kaliumcarbonat und 30 ml Aceton wird 45 Minuten unter Rückfluß erhitzt und anschließend im Wasserstrahlvakuum eingeengt. Der Rückstand wird mit 1 N-Salzsäure und Essigsäureethylester geschüttelt und im Wasserstrahlvakuum eingeengt. Der verbleibende Rückstand wird säulenchromatografisch (Kieselgel, Hexan/-Essigsäureethylester, Vol. 4/1) gereinigt.

Man erhält 2,0 g (69 % der Theorie) 3-[4-Brom-2-fluor-5-(1-methyl-2-oxo-propoxy)-phenyl]-1-methyl-6-trifluormethyl-(1H,3H)-pyrimidin-2,4-dion vom Schmelzpunkt 119°C.

### Vorstufen zu Beispiel (II-1):

### Vorstufe 1

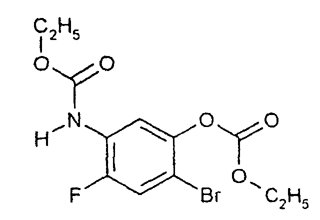

23,9 g (0,22 Mol) Chlorameisensäure-ethylester werden unter Rühren zu einer auf 0°C abgekühlten Mischung aus 20 g (0,97 Mol) 4-Brom-2-fluor-5-hydroxy-anilin, 17,4 g (0,22 Mol) Pyridin und 200 ml Methylenchlorid tropfenweise gegeben und die Reaktionsmischung wird dann noch eine Stunde bei 0°C bis 5°C gerührt. Anschließend wird mit 1 N-Salzsäure geschüttelt, die organische Phase abgetrennt, mit Natriumsulfat getrocknet und filtriert. Das Filtrat wird im Wasserstrahlvakuum eingeengt, der Rückstand mit Petrolether digeriert und das kristallin erhaltene Produkt durch Absaugen isoliert.

Man erhält 30,4 g (90 % der Theorie) N-(4-Brom-2-fluor-5-ethoxycarbonyloxyphenyl)-O-ethyl-carbamat vom Schmelzpunkt 79°C.

### Vorstufe 2

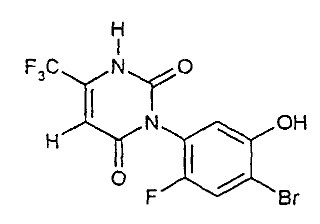

Eine Mischung aus 22 g (0,87 Mol) 3-Amino-4,4,4-trifluor-crotonsäure-ethylester, 30,4 g (0,87 Mol) N-(4-Brom-2-fluor-5-ethoxycarbonyloxy-phenyl)-O-ethyl-carbamat, 4,7 g Natriumhydrid und 50 ml N,N-Dimethyl-formamid wird zwei Stunden auf 125°C erhitzt. Nach Abkühlen wird mit 1 N-Salzsäure/Essigsäureethylester geschüttelt, die organische Phase abgetrennt, mit Natriumsulfat getrocknet und filtriert. Das Filtrat wird im Wasserstrahlvakuum eingeengt und der Rückstand säulenchromatografisch (Kieselgel, Chloroform/Essigsäureethylester, Vol. 7/3) gereinigt.

Man erhält 12,6 g (37 % der Theorie) 3-(4-Brom-2-fluor-5-hydroxy-phenyl)-6-trifluormethyl-(1H,3H)-pyrimidin-2,4-dion als amorphes Produkt.

### Vorstufe 3

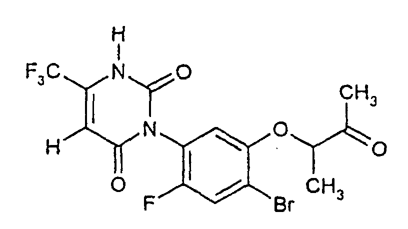

Eine Mischung aus 3,0 g (8,13 mMol) 3-(4-Brom-2-fluor-5-hydroxy-phenyl)-6-trifluormethyl-(1H,3H)-pyrimidin-2,4-dion, 1,06 g (10 mMol) 3-Chlor-2-butanon, 2,34 g Kaliumcarbonat und 50 ml Acetonitril wird 3 Stunden unter Rückfluß erhitzt und anschließend im Wasserstrahlvakuum eingeengt. Der Rückstand wird mit 1 N-Salzsäure/Petrolether verrührt und das kristallin angefallene Produkt durch Absaugen isoliert.

Man erhält 3,1 g (87 % der Theorie) 3-[4-Brom-2-fluor-5-(1-methyl-2-oxo-propoxy)-phenyl]-6-trifluormethyl-(1H,3H)-pyrimidin-2,4-dion vom Schmelzpunkt 183°C.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

In diesem Test zeigen bei Aufwandmengen von 60 bis 125 g/ha beispielsweise die Verbindungen gemäß Herstellungsbeispiel 1, 2, 3 und 4 bei teilweise guter Verträglichkeit gegenüber Kulturpflanzen, wie z.B. Baumwolle (0 %), sehr starke Wirkung gegen Unkräuter wie Echinochloa (100 %), Setaria (100 %), Abutilon (100 %), Amaranthus (100 %), Galium (100 %), Solanum (100 %), Alopecurus (100 %) sowie Sorghum (100 %).

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

In diesem Test zeigen bei Aufwandmengen von 15 g bis 60 g/ha beispielsweise die Verbindungen gemäß Herstellungsbeispiel 1, 2, 3 und 4 bei teilweise guter Verträglichkeit gegenüber Kulturpflanzen, wie z.B. Weizen (0 %), sehr starke Wirkung gegen Unkräuter wie Abutilon (100 %), Datura (100%), Solanum (100 %), Viola (100%), Echinochloa (80-100%), Setaria (80-100%), Sorhum (70-100%), Amaranthus (100 %), Ipomoea (100 %) sowie Veronica (100 %).

## Patentansprüche

1. Substituierte Iminoalkoxy-phenyluracile der Formel (I) 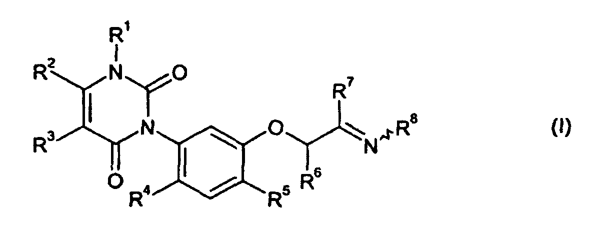 in welcher
R¹ für Wasserstoff, Amino oder gegebenenfalls durch Cyano, Fluor, Chlor oder C₁-C₄-Alkoxy substituiertes C₁-C₆-Alkyl steht,
R² für Carboxy, Cyano, Carbamoyl, Thiocarbamoyl oder für jeweils gegebenenfalls durch Cyano, Fluor, Chlor oder C₁-C₄-Alkoxy substituiertes C₁-C₄-Alkyl oder C₁-C₄-Alkoxycarbonyl steht,
R³ für Wasserstoff, Fluor, Chlor, Brom oder gegebenenfalls durch Fluor oder Chlor substituiertes C₁-C₄-Alkyl steht,
R⁴ für Wasserstoff, Cyano, Thiocarbamoyl, Fluor, Chlor oder Brom steht,
R⁵ für Cyano, Thiocarbamoyl, Fluor, Chlor oder Brom steht,
R⁶ für Wasserstoff oder gegebenenfalls durch Cyano, Fluor, Chlor oder C₁-C₄-Alkoxy substituiertes C₁-C₆-Alkyl steht,
R⁷ für gegebenenfalls durch Cyano, Fluor, Chlor oder C₁-C₄-Alkoxy substituiertes C₁-C₆-Alkyl steht, und
R⁸ für Hydroxy, für Amino oder für jeweils gegebenenfalls durch Carboxy, Cyano, Halogen, C₁-C₄-Alkoxy oder C₁-C₄-Alkoxycarbonyl substituiertes Alkyl, Alkoxy, Alkylamino, Dialkylamino, Alkylcarbonylamino, Alkoxycarbonylamino oder Alkylsulfonylamino mit jeweils 1 bis 6 Kohlenstoffatomen steht,
R⁸ weiterhin für jeweils gegebenenfalls durch Halogen substituiertes Alkenyl, Alkinyl, Alkenyloxy oder Alkinyloxy mit jeweils 3 bis 6 Kohlenstoffatomen steht, oder
R⁸ weiterhin für jeweils gegebenenfalls durch Nitro, Cyano, Carboxy, Halogen, C₁-C₄-Alkyl, C₁-C₄-Halogenalkyl, C₁-C₄-Alkoxy oder C₁-C₄-Halogenalkoxy substituiertes Aryl, Aryloxy, Arylamino, Arylcarbonylamino, Arylsulfonylamino, Arylalkyl, Arylalkylamino, Arylalkylcarbonylamino oder Arylalkylsulfonylamino mit jeweils 6 oder 10 Kohlenstoffatomen im Arylteil und gegebenenfalls 1 bis 4 Kohlenstoffatomen im Alkylteil steht.

2. Substituierte Iminoalkoxy-phenyluracile der Formel (I) gemäß Anspruch 1, **dadurch gekennzeichnet, dass**
R¹ für Wasserstoff, für Amino oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
R² für Carboxy, Cyano, Carbamoyl, Thiocarbamoyl oder für jeweils gegebenenfalls durch Cyano, Fluor, Chlor, Methoxy oder Ethoxy substituiertes Methyl, Ethyl, Methoxycarbonyl oder Ethoxycarbonyl steht,
R³ für Wasserstoff, Fluor, Chlor, Brom oder für jeweils gegebenenfalls durch Fluor oder Chlor substituiertes Methyl oder Ethyl steht,
R⁴ für Wasserstoff, Fluor oder Chlor steht,
R⁵ für Cyano, Thiocarbamoyl, Chlor oder Brom steht,
R⁶ für Wasserstoff oder für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
R⁷ für jeweils gegebenenfalls durch Cyano, Fluor, Chlor, Methoxy oder Ethoxy substituiertes Methyl, Ethyl, n- oder i-Propyl, n-, i-, s- oder t-Butyl steht, und
R⁸ für Hydroxy, für Amino, oder für jeweils gegebenenfalls durch Carboxy, Cyano, Fluor, Chlor, Methoxy, Ethoxy, n- oder i-Propoxy, Methoxycarbonyl, Ethoxycarbonyl, n- oder i-Propoxycarbonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Acetylamino, Propionylamino, Methoxycarbonylamino, Ethoxycarbonylamino, Methylsulfonylamino oder Ethylsulfonylamino steht, oder
R⁸ weiterhin für jeweils gegebenenfalls durch Fluor, Chlor oder Brom substituiertes Propenyl, Butenyl, Propinyl, Butinyl, Propenyloxy, Butenyloxy, Propinyloxy oder Butinyloxy steht, oder
R⁸ weiterhin für jeweils gegebenenfalls durch Nitro, Cyano, Carboxy, Fluor, Chlor, Brom, Methyl, Ethyl, n- oder i-Propyl, n-, i-, s- oder t-Butyl, Trifluor-methyl, Methoxy, Ethoxy, n- oder i-Propoxy, n-, i-, s-oder t-Butoxy, Difluormethoxy, Trifluormethoxy substituiertes Phenyl, Phenoxy, Phenylamino, Benzoylamino, Phenylsulfonylamino, Benzyl, Benzylamino, Benzylcarbonylamino oder Benzylsulfonylamino steht.

3. Verfahren zur Herstellung substituierter iminoalkoxy-phenyluracile der allgemeinen Formel (I) 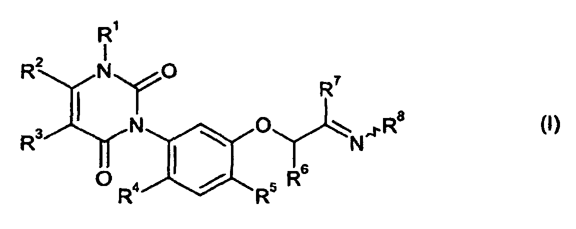 in welcher
R¹, R², R³, R⁴, R⁵, R⁶, R⁷und R⁸ die in Anspruch 1 oder 2 genannten Bedeutungen haben,
**dadurch gekennzeichnet, daß** man
substituierte Oxoalkoxy-phenyluracile der allgemeinen Formel (II) 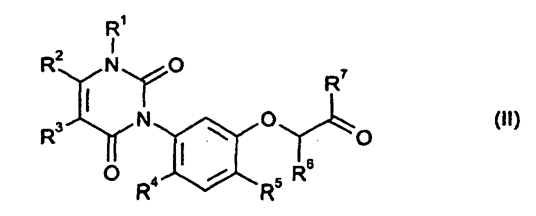 in welcher
R¹, R², R³, R⁴, R⁵, R⁶ und R⁷ die in Anspruch 1 oder 2 angegebenen Bedeutungen haben,
mit Aminoverbindungen der allgemeinen Formel (III)
H₂N-R⁸ (III)
in welcher
R⁸ die in Anspruch 1 oder 2 angegebene Bedeutung hat,
- oder mit Säureaddukten von Verbindungen der allgemeinen Formel (III) -
umsetzt.

4. Herbizide Mittel, **gekennzeichnet durch** einen Gehalt an mindestens einem substituierten Iminoalkoxy-phenyluracil der Formel (I) gemäß Anspruch 1 oder 2.

5. Verfahren zur Bekämpfung von unerwünschten Pflanzen, **dadurch gekennzeichnet, daß** man substituierte Iminoalkoxy-phenyluracile der Formel (I) gemäß Anspruch 1 oder 2 auf unerwünschte Pflanzen und/oder ihren Lebensraum einwirken läßt.

6. Verwendung von substituierten Iminoalkoxy-phenyluracilen der Formel (I) gemäß Anspruch 1 oder 2 zur Bekämpfung von unerwünschten Pflanzen.

7. Verfahren zur Herstellung von herbiziden Mitteln, **dadurch gekennzeichnet, daß** man substituierte Iminoalkoxy-phenyluracile der Formel (I) gemäß Anspruch 1 oder 2 mit Streckmitteln und/oder oberflächenaktiven Substanzen vermischt.

## Claims

1. Substituted iminoalkoxy-phenyluracils of the formula (I) 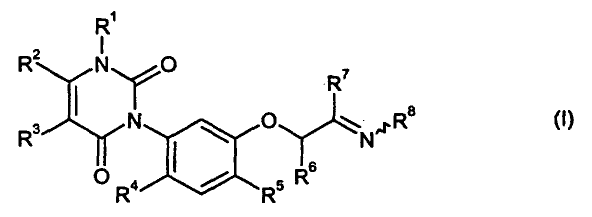 in which
R¹ represents hydrogen, amino or optionally cyano-, fluorine-, chlorine- or C₁-C₄-alkoxy-substituted C₁-C₆-alkyl,
R² represents carboxyl, cyano, carbamoyl, thiocarbamoyl or represents in each case optionally cyano-, fluorine-, chlorine- or C₁-C₄-alkoxy-substituted C₁-C₄-alkyl or C₁-C₄-alkoxycarbonyl,
R³ represents hydrogen, fluorine, chlorine, bromine, or optionally fluorine- or chlorine-substituted C₁-C₄-alkyl,
R⁴ represents hydrogen, cyano, thiocarbamoyl, fluorine, chlorine or bromine,
R⁵ represents cyano, thiocarbamoyl, fluorine, chlorine or bromine,
R⁶ represents hydrogen or optionally cyano-, fluorine-, chlorine- or C₁-C₄-alkoxy-substituted C₁-C₆-alkyl,
R⁷ represents optionally cyano-, fluorine-, chlorine- or C₁-C₄-alkoxy-substituted C₁-C₆-alkyl, and
R⁸ represents hydroxyl, represents amino or represents in each case optionally carboxyl-, cyano-, halogen-, C₁-C₄-alkoxy- or C₁-C₄-alkoxycarbonyl-substituted alkyl, alkoxy, alkylamino, dialkylamino, alkylcarbonylamino, alkoxycarbonylamino or alkylsulphonylamino having in each case 1 to 6 carbon atoms,
R⁸ furthermore represents in each case optionally halogen-substituted alkenyl, alkynyl, alkenyloxy or alkynyloxy having in each case 3 to 6 carbon atoms, or
R⁸ furthermore represents in each case optionally nitro-, cyano-, carboxyl-, halogen-, C₁-C₄-alkyl-, C₁-C₄-haloalkyl-, C₁-C₄-alkoxy- or C₁-C₄-haloalkoxy-substituted aryl, aryloxy, arylamino, arylcarbonylamino, arylsulphonylamino, arylalkyl, arylalkylamino, arylalkylcarbonylamino or arylalkylsulphonylamino having in each case 6 or 10 carbon atoms in the aryl moiety and, if appropriate, 1 to 4 carbon atoms in the alkyl moiety.

2. Substituted iminoalkoxy-phenyluracils of the formula (I) according to Claim 1, **characterized in that**
R¹ represents hydrogen, represents amino or represents in each case optionally cyano-, fluorine-, chlorine-, methoxy- or ethoxy-substituted methyl, ethyl, n- or i-propyl, n-, i-, s- or t-butyl,
R² represents carboxyl, cyano, carbamoyl, thiocarbamoyl or represents in each case optionally cyano-, fluorine-, chlorine-, methoxy- or ethoxy-substituted methyl, ethyl, methoxycarbonyl or ethoxycarbonyl,
R³ represents hydrogen, fluorine, chlorine, bromine or represents in each case optionally fluorine- or chlorine-substituted methyl or ethyl,
R⁴ represents hydrogen, fluorine or chlorine,
R⁵ represents cyano, thiocarbamoyl, chlorine or bromine,
R⁶ represents hydrogen or represents in each case optionally cyano-, fluorine-, chlorine-, methoxy- or ethoxy-substituted methyl, ethyl, n- or i-propyl, n-, i-, s- or t-butyl,
R⁷ represents in each case optionally cyano-, fluorine-, chlorine-, methoxy- or ethoxy-substituted methyl, ethyl, n- or i-propyl, n-, i-, s-or t-butyl, and
R⁸ represents hydroxyl, represents amino, or represents in each case optionally carboxyl-, cyano-, fluorine-, chlorine-, methoxy-, ethoxy-, n- or i-propoxy-, methoxycarbonyl-, ethoxycarbonyl-, n- or i-propoxycarbonyl-substituted methyl, ethyl, n- or i-propyl, n-, i-, s- or t-butyl, methoxy, ethoxy, n- or i-propoxy, n-, i-, s- or t-butoxy, methylamino, ethylamino, n- or i-propylamino, n-, i-, s- or t-butylamino, dimethylamino, diethylamino, acetylamino, propionylamino, methoxycarbonylamino, ethoxycarbonylamino, methylsulphonylamino or ethylsulphonylamino, or
R⁸ furthermore represents in each case optionally fluorine-, chlorine- or bromine-substituted propenyl, butenyl, propynyl, butynyl, propenyloxy, butenyloxy, propynyloxy or butynyloxy, or
R⁸ furthermore represents in each case optionally nitro-, cyano-, carboxyl-, fluorine-, chlorine-, bromine-, methyl-, ethyl-, n- or i-propyl-, n-, i-, s- or t-butyl-, trifluoromethyl-, methoxy-, ethoxy-, n-or i-propoxy-, n-, i-, s- or t-butoxy-, difluoromethoxy-, trifluoromethoxy-substituted phenyl, phenoxy, phenylamino, benzoylamino, phenylsulphonylamino, benzyl, benzylamino, benzylcarbonylamino or benzylsulphonylamino.

3. Process for preparing substituted iminoalkoxy-phenyluracils of the general formula (I) 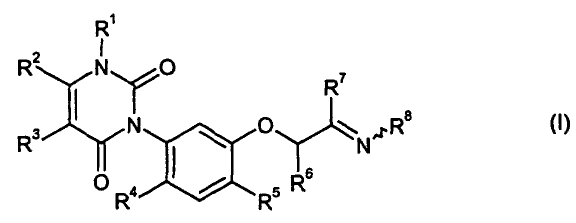 in which
R¹, R², R³, R⁴, R⁵, R⁶, R⁷ and R⁸ are as defined in Claim 1 or 2,
**characterized in that**
substituted oxoalkoxy-phenyluracils of the general formula (II) 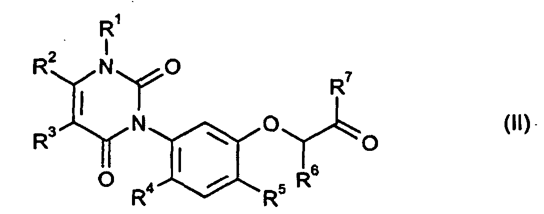 in which
R¹, R², R³, R⁴, R⁵, R⁶ and R⁷ are as defined in Claim 1 or 2
are reacted with amino compounds of the general formula (III)
H₂N-R⁸ (III)
in which
R⁸ is as defined in Claim 1 or 2
- or with acid adducts of compounds of the general formula (III).

4. Herbicidal compositions, **characterized in that** they comprise at least one substituted iminoalkoxy-phenyluracil of the formula (I) according to Claim 1 or 2.

5. Method for controlling undesirable plants, **characterized in that** substituted iminoalkoxy-phenyluracils of the formula (I) according to Claim 1 or 2 are allowed to act on undesirable plants and/or their habitat.

6. Use of substituted iminoalkoxy-phenyluracils of the formula (I) according to Claim 1 or 2, for controlling undesirable plants.

7. Process for preparing herbicidal compositions, **characterized in that** substituted iminoalkoxy-phenyuracils of the formula (I) according to Claim 1 or 2 are mixed with extenders and/or surfactants.

## Revendications

1. Iminoalkoxyphényluraciles substitués de formule (I) 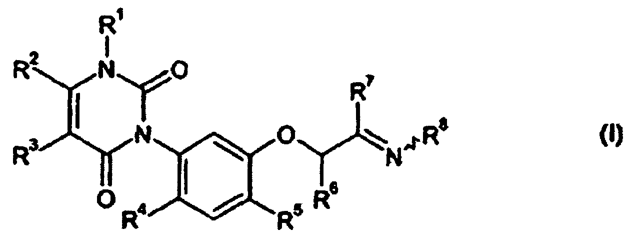 dans laquelle
R¹ représente l'hydrogène, un groupe amino ou un reste alkyle en C₁ à C₆ éventuellement substitué par un radical cyano, fluoro, chloro ou alkoxy en C₁ à C₆,
R² représente un groupe carboxy, cyano, carbamoyle, thiocarbamoyle ou un reste alkyle en C₁ à C₄ ou (alkoxy en C₁ à C₄)carbonyle, chacun éventuellement substitué par un radical cyano, fluoro, chloro ou alkoxy en C₁ à C₄,
R³ représente l'hydrogène, le fluor, le chlore, le brome ou un reste alkyle en C₁ à C₄ éventuellement substitué par du fluor ou du chlore,
R⁴ représente l'hydrogène, un groupe cyano, thiocarbamoyle, le fluor, le chlore ou le brome,
R⁵ représente un groupe cyano, thiocarbamoyle, le fluor, le chlore ou le brome,
R⁶ représente l'hydrogène ou un reste alkyle en C₁ à C₆ éventuellement substitué par un radical cyano, fluoro, chloro ou alkoxy en C₁ à C₄,
R⁷ représente un reste alkyle en C₁ à C₆ éventuellement substitué par un radical cyano, fluoro, chloro ou alkoxy en C₁ à C₄, et
R⁸ représente un groupe hydroxy, un groupe amino ou un reste alkyle, alkoxy, alkylamino, dialkylamino, alkylcarbonylamino, alkoxycarbonylamino ou alkylsulfonylamino ayant chacun 1 à 6 atomes de carbone et chacun éventuellement substitué par un radical carboxy, cyano, halogéno, alkoxy en C₁ à C₄ ou (alkoxy en C₁ à C₄)carbonyle,
R⁸ représente en outre un reste alcényle, alcynyle, alcényloxy ou alcynyloxy ayant chacun 3 à 6 atomes de carbone et chacun étant éventuellement substitué par un halogène, ou bien
R⁸ représente en outre un reste aryle, aryloxy, arylamino, arylcarbonylamino, arylsulfonylamino, arylalkyle, arylalkylamino, arylalkylcarbonylamino ou arylalkylsulfonylamino ayant chacun 6 ou 10 atomes de carbone dans la partie aryle et, le cas échéant, 1 à 4 atomes de carbone dans la partie alkyle, et chacun étant éventuellement substitué par un radical nitro, cyano, carboxy, halogéno, alkyle en C₁ à C₄, halogénalkyle en C₁ à C₄, alkoxy en C₁ à C₄ ou halogénalkoxy en C₁ à C₄.

2. Iminoalkoxyphényluraciles substitués de formule (I) suivant la revendication 1, **caractérisés en ce que**
R¹ représente l'hydrogène, un groupe amino ou un reste méthyle, éthyle, n-propyle, isopropyle, n-butyle, isobutyle, sec.-butyle ou tertiobutyle, chacun éventuellement substitué par un radical cyano, fluoro, chloro, méthoxy ou éthoxy,
R² représente un groupe carboxy, cyano, carbamoyle, thiocarbamoyle ou un reste méthyle, éthyle, méthoxycarbonyle ou éthoxycarbonyle, chacun éventuellement substitué par un radical cyano, fluoro, chloro, méthoxy ou éthoxy,
R³ représente l'hydrogène, le fluor, le chlore, le brome ou un reste méthyle ou éthyle, chacun éventuellement substitué par du fluor ou du chlore,
R⁴ représente l'hydrogène, le fluor ou le chlore,
R⁵ représente un groupe cyano, thiocarbamoyle, le chlore ou le brome,
R⁶ représente l'hydrogène ou un reste méthyle, éthyle, n-propyle, isopropyle, n-butyle, isobutyle, sec.-butyle ou tertiobutyle, chacun éventuellement substitué par un radical cyano, fluoro, chloro, méthoxy ou éthoxy,
R⁷ représente un radical méthyle, éthyle, n-propyle, isopropyle, n-butyle, isobutyle, sec.-butyle ou tertiobutyle, chacun éventuellement substitué par un radical cyano, fluoro, chloro, méthoxy ou éthoxy, et
R⁸ représente un groupe hydroxy, un groupe amino ou un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylamino, éthylamino, n-propylamino, isopropylamino, n-butylamino, isobutylamino, sec.-butylamino, tertiobutylamino, diméthylamino, diéthylamino, acétylamino, propionylamino, méthoxycarbonylamino, éthoxycarbonylamino, méthylsulfonylamino ou éthylsulfonylamino, chacun éventuellement substitué par un radical carboxy, cyano, fluoro, chloro, méthoxy, éthoxy, n-propoxy, isopropoxy, méthoxycarbonyle, éthoxycarbonyle, n-propoxycarbonyle ou isopropoxycarbonyle, ou bien
R⁸ représente en outre un reste propényle, butényle, propynyle, butynyle, propényloxy butényloxy, propynyloxy ou butynyloxy, chacun éventuellement substitué par du fluor, du chlore ou du brome, ou bien
R⁸ représente en outre un reste phényle, phénoxy, phénylamino, benzoylamino, phénylsulfonylamino, benzyle, benzylamino, benzylcarbonylamino ou benzylsulfonylamino, chacun éventuellement substitué par un radical nitro, cyano, carboxy, fluoro, chloro, bromo, méthyle, éthyle, n-propyle, isopropyle, n-butyle, isobutyle, sec.-butyle, tertiobutyle, trifluorométhyle, méthoxy, éthoxy, n-propoxy, isopropoxy, n-butoxy, isobutoxy, sec.-butoxy, tertiobutoxy, difluorométhoxy ou trifluorométhoxy.

3. Procédé de production d'iminoalkoxyphényluraciles substitués de formule générale (I) 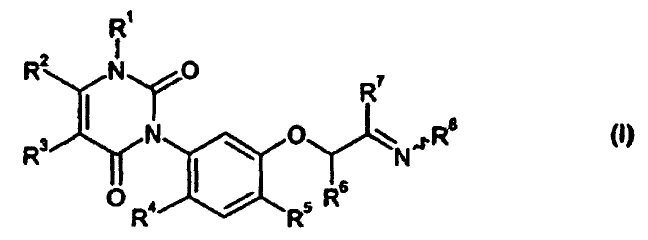 dans laquelle
R¹, R², R³, R⁴, R⁵, R⁶, R⁷ et R⁸ ont les définitions mentionnées dans la revendication 1 ou 2,
**caractérisé en ce qu'**on fait réagir des oxoalkoxyphényluraciles substitués de formule générale (II) 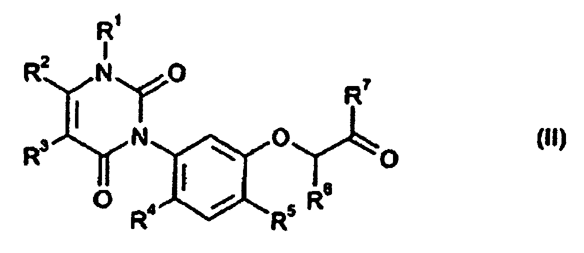 dans laquelle
R¹, R², R³, R⁴, R⁵, R⁶ et R⁷ ont les définitions indiquées dans la revendication 1 ou 2,
avec des composés aminés de formule générale (III)
**H**_{**2**}**N-R**^{**8**} **(III)**
dans laquelle
R⁸ a la définition indiquée dans la revendication 1 ou 2,
- ou avec des sels d'addition d'acides de composés de formule générale (III).

4. Compositions herbicides, **caractérisées par** une teneur en au moins un iminoalkoxyphényluracile substitué de formule (II) selon la revendication 1 ou 2.

5. Procédé de lutte contre des plantes indésirables, **caractérisé en ce qu'**on fait agir des iminoalkoxyphényluraciles substitués de formule (I) suivant la revendication 1 ou 2 sur des plantes indésirables et/ou sur leur milieu.

6. Utilisation d'iminoalkoxyphényluraciles substitués de formule (I) suivant la revendication 1 ou 2 dans la lutte contre des plantes indésirables.

7. Procédé de préparation de compositions herbicides, **caractérisé en ce qu'**on mélange des iminoalkoxyphényluraciles substitués de formule (I) suivant la revendication 1 ou 2 avec des diluants et/ou des agents tensioactifs.
